# EUROPÄISCHE PATENTANMELDUNG

(11) **EP 2 292 255 A2**
(43) Veröffentlichungstag der Anmeldung: **09.03.2011**
(21) Anmeldenummer: 10014814.7
(22) Anmeldetag: 18.12.2003
(51) Int. Cl.: A61K 38/36, A61K 38/37

(54) **Stabile therapeutische Proteine**

(30) Priorität: 18.12.2002 AT 18902002
(62) Teilanmeldung aus: 03781990.1
(71) Anmelder: Bio & Bio Licensing SA, 1724 Luxembourg (LU)
(72) Erfinder: Eibl, Johann, 1180 Wien (AT)
(74) Vertreter: Schwarz & Partner

(57) **Zusammenfassung**

Aus virussicheren, pharmazeutischen Wirkstoffzubereitungen hergestellte, lagerfähige Arzneimittel, die ein aus Plasma oder gentechnologisch gewonnenes, intaktes, therapeutisches Protein bzw. mehrere solche Proteine als aktive pharmazeutische Substanz bzw. Substanzen enthalten, wobei in den Wirkstoffzubereitungen weder freie, noch an ihre Substrate gebundene, aktive Enzyme, insbesondere Proteasen, vorhanden sind, die gegen das bzw. die vorhandene(n) therapeutische(n) Protein(e) wirken.

## Beschreibung

### Einleitung

Therapeutische Proteine sind seit über 100 Jahren in medizinischer Verwendung und nehmen weiter an medizinischem Interesse und Bedeutung zu. Vergleiche mit anderen, aktiven pharmazeutischen Substanzen ergeben, dass therapeutische Proteine als Eiweißstoffe eine wesentlich geringere Stabilität und einen höheren Grad an Verunreinigungen aufweisen. Auch weisen therapeutische Proteine im Vergleich zu anderen pharmazeutischen Wirksubstanzen eine relativ hohe Empfindlichkeit gegenüber verschiedenen chemischen und physikalischen Faktoren auf, insbesondere gegen Enzyme wie Proteasen, die Peptidbindungen spalten. Es sind daher alle Maßnahmen, die bei der Gewinnung, Reinigung und Lagerung intakter therapeutischer Proteine zur Erhaltung ihrer Integrität und Stabilität beitragen, von großer ökonomischer Bedeutung.

Veränderungen therapeutischer Proteine - und das trifft auf alle Proteine zu - können durch intramolekulare Umlagerung oder durch chemische Umsetzungen, wie Spaltung oder Bildung kovalenter Bindungen, erfolgen. Ein Enzym, das ein therapeutisches Protein als sein Substrat erkennt, kann dasselbe weitgehend verändern, und soweit es sich um eine Protease handelt, eine oder mehrere Spaltung(en) in der Peptidkette des Proteins verursachen. Bereits in den Ausgangsmaterialien zur Herstellung von sowohl natürlichen als auch gentechnologisch gewonnenen therapeutischen Proteinen können solche Enzyme wie auch ihre Vorstufen, die als Proenzyme, oder im Fall von Proteasen, auch als Zymogene bezeichnet werden, enthalten sein. Enzyme, die auf Proteine einwirken, sind in den meisten Fällen selbst Proteine und können sowohl frei als auch bereits an ihre Substrate gebunden vorliegen. Ebenso können Zymogene als Substrate vorliegen oder bereits mit ihren spezifischen Enzymen, die sie in Proteasen überführen, verbunden sein.

Enzymatische Einwirkungen auf therapeutische Proteine während ihrer Herstellung und Lagerung, beginnend vom Ausgangsprodukt bis zum Endprodukt, können zu hohen Verlusten und labilen Endprodukten führen (Anderson et al. 1986; Eaton et al. 1986; Brummel et al. 1999).

Bereits in vielen Ausgangsmaterialien sind Enzyme vorhanden, die auf therapeutische Proteine einwirken, ebenso deren Proenzyme und Prokofaktoren, die während des Reinigungsvorganges durch Aktivierung zur weiteren Bildung von solchen Enzymen führen. Prokofaktoren sind ebenfalls Proteine, die durch Einwirkung von Proteasen zu Kofaktoren abgebaut werden, selbst aber keine Enzyme sind. Kofaktoren steigern spezifisch bestimmte Proteaseaktivitäten um ein Vielfaches.

Solche Enzyme, Proenzyme, Kofaktoren oder Prokofaktoren können selbst als therapeutische Proteine zur Anwendung gelangen, vorausgesetzt, dass durch Inaktivierungs- bzw. Abreinigungsvorgänge virale Krankheitserreger, wie sie im Blut oder gentechnologisch gewonnenen Biomassen vorkommen können, im Zuge der Herstellung der pharmazeutisch aktiven Substanz inaktiviert bzw. abgereichert wurden.

Die Blutgerinnungskaskade ist eines der bestbekannten Enzymsysteme. Sie wird durch einen Enzymkofaktor, den lipidhältigen Tissue Factor, der die Enzymaktivität des Gerinnungsfaktors Vlla um ein Vielfaches erhöht, ausgelöst. Der Tissue Factor kommt normalerweise nur in Zellen vor und kann dadurch mit dem Gerinnungsfaktor Vlla nicht in Wechselwirkung treten. Durch pathologische oder traumatische Ereignisse kann der Tissue Factor an die Oberfläche von Tissue-Factor-hältigen Zellen gelangen, bzw. aus diesen Zellen austreten. Mit den im Blut immer vorhandenen geringen Mengen Faktor Vlla kann dann die Enzymkaskade ausgelöst werden. Der Endpunkt dieser Gerinnungskaskade ist das Enzym Thrombin, das so über den extrinsic und common Pathway entsteht und Fibrinogen in Fibrin überführt. Fibrinogen, das auch als therapeutisches Protein verwendet werden kann, wird von Thrombin in Fibrinmonomer und Fibrinopeptide gespalten. Fibrinmonomer polymerisiert zu Fibrinsträngen und schließlich zu einem Netzwerk, das im Wundbett zu einem Wundverschluss und damit zur Beendigung einer Blutung führen kann. Fibrinogen ist weder Enzym, noch besitzt es Kofaktoreneigenschaften (Lawson et al. 1994; Hemker 2002; Mann et al. 2002).

Thrombin wirkt aber nicht nur auf Fibrinogen enzymatisch ein, sondern führt auch unter anderem das Proenzym Gerinnungsfaktor XIII in das Enzym Gerinnungsfaktor XIIIa über. Dieses bewirkt als Transglutaminase durch Quervernetzungen der Fibrinstränge im dreidimensionalen Fibringerüst eine erhöhte biomechanische Stabilität des gebildeten Fibrinnetzwerkes und ebenso einen Schutz vor enzymatischem Abbau. Im durch die Thrombineinwirkung geronnenen Blut, bzw. Plasma, kommt es zu weiteren wichtigen enzymatischen Vorgängen, insbesondere der Thrombinbildung aus dem Proenzym Prothrombin, dem Gerinnungsfaktor II. Dadurch kommt es zu einer starken Erhöhung der Thrombinkonzentration im geronnenen Blut, die nicht nur die komplette Umwandlung des Gerinnungsfaktors XIII in XIIIa, sondern auch die Aktivierung des Proenzyms TAFI. zum Enzym TAFIa verursacht. Dieses Enzym spaltet von Fibrin ein kurzes Peptid ab, das Rezeptoren für einen fibrinolytischen Enzymkomplex enthält und so der Resistenz von Fibrin gegenüber fibrinolytischen Einflüssen dient. Für diese Vorgänge ist eine relativ hohe Konzentration von Thrombin im bereits geronnenen Blut erforderlich, die durch Aktivierung des im geronnenen Blut vorhandenen Prothrombins generiert werden kann (Siebenlist et al. 2001).

Die stark erhöhte Bildung von Thrombin im Blutgerinnsel selbst kommt nicht mehr über den extrinsic Tenase-Pathway zustande, sondern über den intrinsic Tenase-und den common Pathway. Schon geringe Mengen Thrombin führen in Anwesenheit von Faktor VIII und Faktor IXa und gerinnungsaktivem Phospholipid zur Bildung des intrinsic Tenasekomplexes, der ebenso wie der extrinsic Tenasekomplex, aber 50fach stärker, die Aktivierung durch proteolytische Spaltung des Gerinnungsfaktors X in Xa bedingt, der dann zur Thrombinbildung durch Aktivierung von Prothrombin führt (Mutch et al. 2001).

Bei physiologischen wie auch bei pathophysiologischen Gerinnungsvorgängen bewirken bestimmte zelluläre Phospholipidstrukturen in und an Zellen, insbesondere in Blutplättchen, eine zusätzliche, starke Beschleunigung bestimmter enzymatischer Vorgänge in der Gerinnungskaskade, zusätzlich zu den aktivitätserhöhenden, hochmolekularen Kofaktoren.

### Stand der Technik

Schon bei den bisherigen Methoden zur Gewinnung therapeutischer Proteine wurden Reinigungsbedingungen angewandt, die Enzymeinwirkungen auf die zu gewinnenden Proteine zu minimieren suchten, indem zum Beispiel der Reinigungsvorgang bei möglichst niederen Temperaturen und/oder durch Zusatz von Inhibitoren durchgeführt wurde (Kunicki et al. 1987 und 1992; Johnson et al. 1994 und 1996; Rotblat et al. 1985). Trotzdem erwies sich diese Vorgangsweise insbesondere bei Proteinen, die einer leichten enzymatischen Spaltung unterliegen, als nicht ausreichend. Ebenso wurden die Abreinigung solcher Enzyme, ihrer Proenzyme, Kofaktoren und Prokofaktoren während des Reinigungsverfahrens nicht ausreichend quantitativ verfolgt und somit auch die Nachbildung solcher Enzyme während des gesamten Herstellungsverfahrens nicht genügend berücksichtigt. Viele der bisher gewonnenen therapeutischen Proteine waren aus diesem Grund nur in unzureichender Ausbeute mit veränderter und verminderter biologischer Wirksamkeit sowie mit hoher Labilität, insbesondere bei der erforderlichen Lagerung des fertigen Arzneimittels, gekennzeichnet.

Die bei der Reinigung bisher verwendeten Enzyminhibitoren wiesen eine zu geringe Avidität und/oder Konzentration auf. Dadurch konnten Enzyme, die bereits an ihr Substrat gebunden waren, nur unzureichend neutralisiert werden. Weiters wurde auch nicht darauf geachtet, die Konzentration von Enzyminhibitoren während des Reinigungsvorganges möglichst konstant zu halten.

Gentechnologisch gewonnene, therapeutische Proteine können bereits bei ihrer Expression und Ausschleusung aus Zellen proteolytische Enzyme gebunden haben bzw. solche, die im Kulturmedium vorhanden sind, binden. Ähnliches trifft auch auf therapeutische Plasmaproteine zu. Bei der Gewinnung, Lagerung und Verarbeitung von Plasma können vor allem proteolytische Enzyme gebildet werden, die Plasmaproteine als ihr Substrat erkennen. Dadurch kommt es zunächst zur Bildung von Protease-Substratkomplexen und in weiterer Folge zur enzymatischen Spaltung des Substrats.

Nach einer solchen Komplexbildung mit einem therapeutischen Protein als hochmolekularem Substrat sind Proteasen durch Inhibitoren nicht oder nur mehr geringfügig hemmbar. Durch diese Vorgänge können therapeutische Proteine bereits bei ihrer Herstellung und insbesondere bei der Lagerung weitgehend in ihrer Qualität verändert oder zerstört werden.

Bereits bei dem ersten Fraktionierungsvorgang der Kryopräzipitation von Plasma können enzymbedingte Veränderungen im Kryopräzipitat und im Kryopräzipitatüberstand auftreten, die zu einer schlechteren Ausbeute und Qualitätsminderung der in weiterer Folge des Herstellungsvorganges gewonnenen therapeutischen Proteine führen. Dies trifft besonders für den aus Kryopräzipitat gewinnbaren Gerinnungsfaktor VIII zu. Enzymatische Vorgänge im Kryopräzipitat können auch zur Aktivierung von Gerinnungsfaktor XIII führen, der gemeinsam mit Thrombin für die vermehrte Bildung von Fibrinmonomerkomplexen verantwortlich ist. Fibrinmonomerkomplexe wirken sich nachteilig auf die Qualität des aus Kryopräzipitat gewinnbaren Fibrinogen aus.

Vom Überstand der Kryopräzipitation kann durch Bindung an schwache Anionenaustauscher und Elution mit Salzlösungen ein Gemisch von Gerinnungsfaktoren eluiert werden. Dieses Gemisch enthält im Wesentlichen die Gerinnungsfaktoren II, VII, IX und X und wird als Prothrombinkomplex bezeichnet. Es ist auch möglich, aus dem Prothrombinkomplex einzelne Gerinnungsfaktoren zu isolieren und diese zu einer pharmazeutischen Präparation weiterzuverarbeiten. Der Prothrombinkomplex, zu einer pharmazeutischen Präparation weiterverarbeitet, muss weitere Herstellungsstufen durchlaufen, um eine weitgehende Entfernung von potentiell vorhandenen Viren zu gewährleisten. Diese Herstellungsstufen müssen bei Raumtemperatur oder erhöhter Raumtemperatur durchgeführt werden. Bedingt durch das Ausgangsmaterial sowie durch die Herstellung besteht die Möglichkeit, dass im Prothrombinkomplex vorhandene Gerinnungsfaktoren aktiviert werden, die bei der parenteralen Anwendung des Prothrombinkomplexes zumindestens mitbeteiligt sind an Nebenwirkungen dieses Produkts, die sich in peripheren Verschlüssen, Myokardinfarkten, Lungenembolien oder Schlaganfällen manifestieren können. Es ist daher das Ziel, die im Prothrombinkomplex enthaltenen Gerinnungsfaktoren so zu gewinnen, dass sie weder bei der Lagerung, noch während des Herstellungsvorganges, noch bei der nachfolgenden Anwendung aktiviert werden.

Auch bei der Gewinnung von Thrombin, Plasminogen und Immunglobulinen, die aus unterschiedlichen Fraktionen des Kryopräzipitations-Überstandes gewonnen werden, spielen Aktivierungen von Zymogenen eine nachteilige Rolle.

So kann Thrombin mit Plasmin, das aus seinem Zymogen, Plasminogen, entsteht, verunreinigt sein und dadurch eine ungenügende hämostatische Wirkung aufweisen. Entsprechend dem Plasmingehalt kommt es zu einer vorzeitigen Lösung des Wundverschlusses und dem Wiederauftreten von Blutungen.

Auch bei der Gewinnung von Immunglobulinen spielen Aktivierungen von Zymogenen eine wichtige Rolle. So wird Immunglobulin G durch Plasmin in ein Fab-Fragment und ein Fc-Stück gespalten. Diese enzymatische Spaltung des Immunglobulins führt bei parenteraler Anwendung des Immunglobulin-Gs zu einer stark verkürzten Halbwertszeit und zu einer Qualitätsminderung der in der Immunglobulin-Präparation enthaltenen Antikörper. Für therapeutische Zwecke ist es oft erforderlich, große Mengen von Immunglobulin G - bis zu 250 g pro Patient - zu verabreichen. Dabei wurde mehrfach über schwere Nebenwirkungen, wie Myokardinfarkte und zentrale Gefäßverschlüsse berichtet. Es ist daher von großem Interesse, Immunglobulin-Präparationen herzustellen, die keine Prokoagulantien enthalten.

Durch Proteaseeinwirkung kann es auch oft zur homomeren und heteromeren Aggregatbildung, die sich bis zur sichtbaren Partikelbildung unlöslicher Aggregate auswirkt, zu Erhöhungen der Viskosität, Verlangsamung der erforderlichen Filtrationsschritte, Ausbeuteverminderung und unbefriedigender Stabilität bei Lagerung der Intermediär- und Fertigprodukte kommen.

### Aufgabenstellung

Die Aufgabenstellung ergibt sich aus der Notwendigkeit, die Bildung von Proteasen und ihre Einwirkung auf therapeutische Proteine bei deren Herstellung und Lagerung weitestgehend zu verhindern, damit unbefriedigende Ausbeuten bei den erforderlichen Reinigungsprozessen sowie Qualitätsminderungen auf Grund von durch enzymatische Vorgänge bedingten Veränderungen ihrer molekularen Struktur vermieden werden. Es ist daher erforderlich, Herstellungsverfahren zur Anwendung zu bringen, die eine Gewinnung und Aufarbeitung des Ausgangsmaterials erlauben, ohne dass es zu enzymbedingten Veränderungen therapeutischer Proteine während des erforderlichen Reinigungsprozesses inklusive der Virusentfernung kommt.

Somit ist es erforderlich, dass bei der Herstellung und Lagerung therapeutischer Proteine diese durch entsprechende Abreinigung und/oder Zusatz von Enzyminhibitoren von allen direkten Einwirkungen von Enzymen, die sie als Substrate erkennen, sowie von indirekten Einwirkungen durch Proenzyme, Kofaktoren und Prokofaktoren geschützt sind.

Es ist auch erforderlich, jene Enzymkonzentrationen zu bestimmen, die noch zu keiner Veränderung eines bestimmten therapeutischen Proteins führen, unter Berücksichtigung der Einwirkungszeit und der sonstigen, für die enzymatische Aktivität wichtigen Bedingungen.

Für die Stabilität von therapeutischen Proteinen ist die Messung von Enzymaktivitäten über längere Zeiträume erforderlich, um aus der akzelerierten Stabilitätsuntersuchung Rückschlüsse auf die Langzeitstabilität machen zu können. Solche akzelerierten Stabilitätsuntersuchungen erstrecken sich meist auf den Zeitraum von mindestens einem Monat.

Da schon sehr geringe Enzymmengen zu Veränderungen therapeutischer Proteine führen können, ist die Entwicklung von entsprechenden Bestimmungsmethoden, die sich über längere Zeiträume erstrecken, erforderlich.

Von Wichtigkeit ist auch die Messung der Inhibitorenmenge, die benötigt wird, um nicht entfernbare Proteasen zu hemmen und diese Hemmung langfristig aufrecht zu erhalten.

### Aufgabenlösung

Die Erfindung betrifft somit aus virussicheren, pharmazeutischen Wirkstoffzubereitungen hergestellte, lagerfähige Arzneimittel, die ein aus Plasma oder gentechnologisch gewonnenes, intaktes, therapeutisches Protein bzw. mehrere therapeutische Proteine als aktive pharmazeutische Substanz bzw. Substanzen enthalten, wobei in den Wirkstoffzubereitungen weder freie, noch an ihre Substrate gebundene, aktive Enzyme, insbesondere Proteasen, in Konzentrationen vorhanden sind, die ein therapeutisches Protein oder therapeutische Proteine in ihrer Sicherheit oder Wirksamkeit mindern.

Sind therapeutische Proteine Zymogene, so dürfen sie keine Proteasen oder aktivierbaren Enzymkaskaden enthalten, die Zymogene zu Proteasen aktivieren.

Therapeutische Proteine sollen nach Möglichkeit keine Proenzyme enthalten, die zu Enzymen aktiviert werden können, die therapeutische Proteine als Substrate erkennen, ebenso keine Kofaktoren und keine Prokofaktoren, die diese Aktivierung fördern.

Die Lösung der Aufgabenstellung wird daher durch konsequent eingehaltene Herstellungs- und Lagerungsbedingungen erreicht, bei denen die Einwirkung eines Enzyms oder mehrerer Enzyme auf therapeutische Proteine, die ihre Substrate sind, weitestgehend unterbunden wird, ebenso wie die Bildung solcher Enzyme aus ihren entsprechenden Proenzymen.

Um enzymbedingte Veränderungen in Ausgangsmaterialien, Zwischenprodukten und Endprodukten zu unterbinden, ist es erforderlich, geringfügige Aktivitäten von Proteasen zu bestimmen, die noch zu keiner Veränderung aktiver therapeutischer Proteine führen. Hierzu sind Bestimmungsmethoden erforderlich, die noch Spuren von Enzymen erfassen können. Am Beispiel 1 der ultrasensitiven Bestimmung von Thrombin wird gezeigt, dass durch Langzeitinkubation mit chromogenen Substraten noch Thrombinmengen zwischen 30 bis 100 µE/Milliliter bestimmbar sind. Dies entspricht einer 300 - 1000 pikomolaren Lösung von Thrombin und einer Menge von 300 bis 1000 Allomolen/ mL.

Erfindungsgemäß wird die Kontrolle einer bestimmten Enzymaktivität so durchgeführt, dass ein empfindliches, niedermolekulares Substrat für die zu bestimmende Protease ausgewählt wird und während des gesamten Herstellungsprozesses eine Parallelprobe denselben Herstellungsbedingungen unterworfen und die enzymatische Spaltung des Substrats in dieser Probe gemessen wird. Die in den Parallelproben verwendeten, niedermolekularen Substrate müssen eine möglichst hohe Stabilität aufweisen, damit sie bei Langzeituntersuchungen verwendet werden können. Geeignete, chromogene oder fluorogene Substrate müssen ausgewählt werden, die maximal von der zu untersuchenden Protease gespalten werden. Die Mengen eines gespaltenen Substrats, die bei der Herstellung, Zwischenlagerung und Lagerung eines therapeutischen Proteins entstehen, können in Katal-Äquivalenten ermittelt und auf das Gewicht oder die Aktivität eines therapeutischen Proteins bezogen werden. Siehe Beispiel 9.

Die Erfindung betrifft ferner Reinigungsverfahren zur Gewinnung therapeutischer Proteine aus Blut oder Biomassen. Die hiefür erforderlichen Hilfsmaterialien können am Ende des Herstellungsprozesses ganz oder teilweise aus den erhaltenen Wirkstoffen entfernt werden. Der Herstellungsprozess wird mit Hilfe der weitgehenden Ausschaltung von Proteaseaktivitäten so geführt, dass mindestens 60%, vorzugsweise 95%, des jeweiligen therapeutischen Proteins, bezogen auf das Ausgangsmaterial, gewonnen werden können.

Erfindungsgemäß können auch zusätzlich atoxische, chaotrope Substanzen eingesetzt werden, die eine Enzym-Substratbindung dissoziieren bzw. eine solche Bindung nicht zustande kommen lassen und dadurch die Trennung von an therapeutische Proteine gebundenen Enzymen, insbesondere Proteasen, verbessern, wobei die chaotropen Substanzen nach dem Trennverfahren wieder entfernt werden.

Durch konsequente Einhaltung tiefer Temperaturen zwischen 0° C bis 4° C, Komplexbindung von Kalziumionen, Anwendung bestimmter anorganischer und organischer Substanzen sowie chaotroper Agenzien, Einstellung von Redoxpotenzialen und Anwendung geeigneter, gelöster oder immobilisierter Enzyminhibitoren werden Enzymaktivitäten so tief wie möglich gehalten. Bei Verwendung löslicher, hochmolekularer Enzyminhibiitoren muss sichergestellt sein, dass sie atoxisch und virussicher sind und bei den verwendeten Konzentrationen therapeutische Proteine in ihrer Wirkung nicht beeinflussen. Siehe Beispiel 10.

Noch vorhandene Restaktivitäten von Enzymen werden während des gesamten Herstellungsvorganges in Proben von allen Zwischenprodukten durch Zusatz empfindlicher, niedermolekularer Substrate für die einzelnen zu verfolgenden Enzyme gemessen. Wenn in einer bestimmten Zwischenstufe die Enzymaktivität die festgelegten Mindestgrenzen übersteigt, wird umgehend die Konzentration des verwendeten Inhibitors, bzw. der verwendeten Inhibitoren erhöht. Siehe Beispiel 13.

Bei Herstellungsstufen, bei denen noch vorhandene Enzymaktivitäten stören, werden geeignete, gelöste oder immobilisierte Enzyminhibitoren in erforderlichem Ausmaß zugesetzt. Gelöste, allogene Inhibitoren werden entfernt oder mit dem therapeutischen Protein zusammen als Wirksubstanz verwendet.

Bei der Herstellung therapeutischer Proteine ist es erforderlich, Virusinaktivierungen und Virusabreicherungen durchzuführen, die in den meisten Fällen bei Raumtemperatur oder etwas darüberliegenden Temperaturen vorgenommen werden müssen. Viruzide Chemikalien werden nach Beendigung des Virusinaktivierungsverfahrens wieder von den therapeutischen Proteinen abgetrennt. Die Virusabreicherung von therapeutischen Proteinen kann z.B. durch Nanofiltration bei Temperaturen zwischen 20° C bis 40° C unter Verwendung von Nanofiltern mit einer Porenweite von 75 nm und 35 nm vorgenommen werden. Um zu einer sicheren Abreicherung aller Viren zu kommen, ist die Anwendung eines 20 nm-Porenfilters und nachfolgend eines 15 nm-Porenfilters erforderlich. Da diese Verfahrensschritte zwischen 20° C und 40° C vorgenommen werden, müssen unerwünschte Enzymaktivitäten durch Zugabe von geeigneten Inhibitoren entsprechend herabgesetzt werden. Siehe Beispiel 10.

Verschiedene proteolytische Aktivitäten nehmen bei Lagerung von Zitratplasma trotz tiefen Lagertemperaturen bei -20° C stark zu. Solche stark erhöhten Enzymaktivitäten sind auch in dem aus diesem Plasma hergestellten Kryopräzipitat und Kryopräzipitatüberstand bestimmbar, wie in Beispiel 1 gezeigt wird. Durch Zusatz von EDTA zu frisch gewonnenem Zitratplasma kann die Kalziumionenaktivität, die mit einer Kalziumelektrode gemessen wird, praktisch vollständig unterbunden werden. Durch diesen Zusatz kann bei Lagerung des Plasmas in tiefgefrorenem Zustand die Bildung proteolytischer Enzyme unterbunden werden. Bei der Lagerung von Kryopräzipitatpaste in tiefgefrorenem Zustand, die aus frisch gewonnenem Plasma hergestellt wird, kommt es zur Aktivierung von Proteasen. Die Bildung proteolytischer Enzyme kann durch Zugabe von EDTA vor dem Einfrieren des Kryopräzipitats verhindert werden. Bei umgehender Aufarbeitung eines Kryopräzipitatüberstandes ist kein weiterer Zusatz von EDTA erforderlich. Bei erwünschter Lagerung des Kryopräzipitatüberstandes in tiefgefrorenem Zustand kann durch Zusatz von geringen Mengen Heparin vor dem Einfrieren die Bildung von Proteasen verhindert werden. Siehe Beispiel 1.

Die Erfindung betrifft auch ein virussicheres Fibrinogen als aktive, pharmazeutische Substanz in Arzneimitteln, das dadurch gekennzeichnet ist, dass es weniger als 4% Fibrinmonomerkomplexe, bezogen auf das Gesamtprotein (Fibrinogen und Fibrinmonomerkomplexe), und weniger als 1 µE Thrombin pro mg Fibrinogen aufweist. Siehe Beispiel 2.

Erfindungsgemäß ist ferner ein in flüssigem Zustand bei Kühlschranktemperatur lagerfähiges, fibrinogenhältiges Arzneimittel, das dadurch gekennzeichnet ist, dass es eine Thrombinaktivität von weniger als 1 µE Thrombin pro mg Fibrinogen und Arginin als chaotropes Agens in einer Konzentration von 0,5% bis 5,0% enthält. Wenn Fibrinogen aus Plasma hergestellt wird, das längere Zeit bei - 20° C gelagert wurde, so kann es 5% oder mehr Fibrinmonomerkomplexe enthalten. Dies führt bei Lagerung im Kühlschrank zu einer Gelbildung. Je nach Gehalt an Fibrinmonomerkomplexen kann diese Gelbildung durch Zusatz von chaotropen Agenzien, z.B. durch Arginin, hintangehalten werden. Siehe Beispiel 3.

Thrombin wird als solches oder gemeinsam mit Fibrinogen zur Sistierung von Blutungen verwendet, da es in genügender Konzentration angewendet, umgehend zur Ausbildung eines hämostatischen Pfropfens führt. Thrombinpräparationen können mit Plasmin verunreinigt sein, und solche Präparationen führen zwar ebenfalls, mit Fibrinogen zusammengebracht, zur Ausbildung eines hämostatischen Pfropfens, jedoch löst sich dieser wegen der Anwesenheit von Plasmin mehr oder weniger rasch auf, wodurch die sistierte Blutung neuerlich beginnen kann. Wie in Beispiel 4 gezeigt wird, gelingt es durch die Abreinigung von Plasminogen und Plasmin, eine Thrombinpräparation herzustellen, die frei von fibrinolytischen Eigenschaften ist.

Immunglobulin G, das als fertige pharmazeutische Präparation sowohl gefriergetrocknet als auch flüssig gelagert werden kann, wird im Wesentlichen zur Behebung eines Antikörpermangels bei Patienten mit Antikörpermangelsyndrom, und neuerdings bei verschiedenen schweren entzündlichen Erkrankungen, insbesondere in der Neurologie, eingesetzt. Die Dosierung bei solchen entzündlichen Erkrankungen kann bis zu dem Zehnfachen der bei Immunmangelerkrankungen verwendeten Menge betragen. Bei dieser Hochdosisbehandlung mit Immunglobulin G können Nebenwirkungen auftreten, die durch periphere, koronare oder zerebrale Gefäßverschlüsse zustande kommen. Aus diesem Grund sollen Immunglobulin-Präparationen weitestgehend frei von Prokoagulanzien sein. Immunglobulin-Präparationen sind im Allgemeinen mit Faktoren der Kontaktphase, wie Kallikrein und aktiviertem Gerinnungsfaktor XI verunreinigt. Durch Zusatz von C₁-Inhibitor und Heparin können diese Enzymaktivitäten weitgehend abgesenkt, bzw. nicht mehr nachweisbar gemacht werden. Siehe Beispiel 5.

Weiterhin kann es durch Einwirkung von Proteasen, vornehmlich Plasmin, auf Immunglobuline zur Aufspaltung in das Fab-Fragment und das Fc-Stück kommen. Dabei tritt eine starke Verminderung der biologischen Wirksamkeit auf, indem die Bioverfügbarkeit zeitlich stark herabgesetzt und die Qualität der in der Immunglobulin-G-Präparation enthaltenen Antikörper gemindert wird. Plasmin bildet sich aus dem in den meisten Immunglobulin-Präparationen enthaltenen Plasminogen während der Herstellung und Lagerung des Produktes. Durch Zusatz gelöster oder immobilisierter Inhibitoren, wie Aprotinin, können sowohl die Plasminbildung als auch die Plasminwirkung unterbunden werden.

Durch wiederholte Salzfällung in Anwesenheit von Harnstoff ist es möglich, fibrinolytische Aktivitäten soweit abzutrennen, dass bei wiederholten Fällungen in der Immunglobulin-Präparation keine Fibrinolyse mehr nachweisbar ist. Siehe Beispiel 5.

Die Erfindung betrifft ferner eine virussichere Faktor-VIII-Zubereitung, die dadurch gekennzeichnet ist, dass sie keine nachweisbare Thrombinaktivität und einen Antithrombingehalt von weniger als 10 arbiträren Antithrombineinheiten pro Einheit Faktor VIII aufweist und pro Einheit Faktor VIII nicht mehr als 0,2 Einheiten Faktor VIIIa enthalten sind.

Der Prothrombinkomplex besteht aus vier Zymogenen, den Gerinnungsfaktoren II, VII, IX und X. Ihre Bildung im Organismus ist Vitamin-K-abhängig. Durch Vitamin-K-Antagonisten, wie z.B. Warfarin, kann die Bildung dieser Gerinnungsfaktoren dosisabhängig vermindert werden, wodurch eine orale Antikoagulansbehandlung in gewünschter Intensität ermöglicht wird. Diese Behandlung ist zur Verhinderung drohender Gefäßverschlüsse erforderlich. Durch parenterale Gabe von Prothrombinkomplex kann die orale Antikoagulansbehandlung, wenn erforderlich, sofort unterbrochen werden. Ebenso kann bei Patienten, deren Grundkrankheit zu einem Prothrombinkomplexmangel führt, durch parenterale Zufuhr von Prothrombinkomplex eine Blutungsneigung sofort aufgehoben werden.

Bei parenteraler Behandlung mit Prothrombinkomplex-Präparaten treten manchmal schwere Nebenwirkungen durch Gefäßverschlüsse auf, die durch Thrombusbildung bewirkt sind. Solche Gefäßverschlüsse werden auf aktivierte Gerinnungsfaktoren, die in Prothrombinkomplexen enthalten sein können, zurückgeführt. Die Aktivierung der im Prothrombinkomplex enthaltenen Gerinnungsfaktoren erfolgt durch den aktivierten Gerinnungsfaktor Xa, der zur Thrombinbildung aus Prothrombin und nachfolgend zur Bildung von Fibrin aus Fibrinogen führt. Die Aktivierung von Faktor Xa kann sowohl über den extrinsic Tenase-Pathway erfolgen, der Gerinnungsfaktor Vlla abhängig ist, als auch noch in stärkerem Ausmaß über den intrinsic Tenase-Pathway, der von den aktivierten Gerinnungsfaktoren IX und VIII abhängig ist.

Erfindungsgemäß wird die Bildung von Faktor Xa über den intrinsic Tenase-Pathway durch vollständige Abreinigung der Gerinnungsfaktoren VIII und VIIIa aus dem Prothrombinkomplex erzielt. Durch Erhaltung eines möglichst hohen Gehalts an plasmatischem TFPI in der Prothrombinkomplex-Präparation wird die Faktor-Xa-Bildung über den extrinsic Tenase-Pathway unterbunden. Siehe Beispiel 7.

Wie in Beispiel 9 gezeigt wird, ist es möglich, proteolytische Enzyme in äußerst niederen Konzentrationen nachzuweisen und zu bestimmen, indem die Inkubationszeiten von Enzym mit chromogenem Substrat um das 100- bis 1000-fache verlängert werden. Durch Auswahl stabiler chromogener Substrate gelingt es, die Wirkung von Proteasen auch bei tiefen Temperaturen nahe dem Gefrierpunkt nachzuweisen und zu bestimmen, wobei Inkubationszeiten um das 1000- bis 10000-fache gegenüber der normalen Inkubationszeit von drei Minuten angewandt werden. Diese Spaltung von chromogenen Substraten über lange Zeit wird auch in Anwesenheit der natürlichen hochmolekularen Substrate der Enzyme nicht vermindert.

Enzymatische Umsatzprodukte, die durch Enzyme in mikro- bis femtomolaren Konzentrationen verursacht werden, können in erfindungsgemäß hergestellten therapeutischen Proteinen durch Massenspektrometrie, HPLC, Gelfiltration und chromatographische, elektrophoretische oder immunologische Methoden nachgewiesen oder bestimmt werden. Siehe Beispiel 12.

Die Enzymaktivitäten können an Proben zur Prozess-Kontrolle bestimmt werden, indem während des gesamten Herstellungsprozesses eine gewählte, chromogene Substratkonzentration, erforderlichenfalls durch ergänzenden chromogenen Substratzusatz, aufrechterhalten wird. Das enzymatisch gespaltene, chromogene Substrat wird als Differenz zwischen dem insgesamt gespaltenen, chromogenen Substrat abzüglich des Substrats in entsprechenden Kontrollen ermittelt. Bei Verwendung von p-Nitroanilin-hältigen, chromogenen Substraten wird mit Hilfe einer p-Nitroanilin-Eichkurve die absolute Menge des gespaltenen, chromogenen Substrats ermittelt.

Um optimale Lagerbedingungen für therapeutische Proteine enthaltende Arzneimittel zu erreichen, kann der Zusatz von Enzyminhibitoren bei der Formulierung des Arzneimittels erforderlich sein. Bei der Anwendung solcher Arzneimittel, die sich über mehrere Stunden erstreckt, muss das flüssige Arzneimittel eine genügend hohe Stabilität bei Raumtemperatur besitzen. Bei nur teilweisem Verbrauch und Aufbewahrung des nicht verbrauchten Arzneimittels im Kühlschrank muss eine Stabilität über etwa drei Tage bei Kühlschranktemperatur gewährleistet sein. Siehe Beispiel 8.

Bevorzugte Ausgestaltungen der Erfindung werden an Hand der folgenden Beispiele näher erläutert.

### Beispiele:

### 1. Gewinnung von proteasearmem Ausgangsmaterial und daraus hergestellten Rohfraktionen zur Herstellung therapeutischer Proteine:

### A. Proteasearmes Plasma:

Frisches Zitratplasma mit einem Gehalt von nicht mehr als 300 µE Thrombin pro mL wird mit soviel einer 1%igen EDTA-Lösung versetzt, dass die Kalziumionenaktivität nicht mehr als die Ionenaktivität einer 10 µM Kalziumchloridlösung beträgt. Messungen wurden mit einer Kalziumelektrode Typ 15 220 3000 der Firma Mettler Toledo durchgeführt. Die Messketten-Spannung wurde in mV abgelesen, und es wurde eine Eichkurve im oberen und unteren Messbereich bei 25° C erstellt. Die Komplexbindung von Kalziumionen durch EDTA wird bei Zugabe von EDTA durch Verringerung der Messketten-Spannung gemessen. Messketten-Spannungen, die unter -15 mV lagen, wurden nicht mehr berücksichtigt. Als unterster Messbereich wurden Messketten-Spannungen, die einer 5 - 10 µM Kalziumchloridlösung entsprechen, nicht mehr berücksichtigt. Das mit EDTA-Lösung versetzte Plasma wird ebenfalls eingefroren, die Kalziumionenaktivität in aufgetauten Proben in 30-tägigen Abständen bestimmt und ebenso die Thrombin- und thrombinartige Aktivität mit chromogenem Substrat S-2238. In dem mit EDTA versetzten Plasma kommt es zu keiner signifikanten Steigerung der Enzymaktivität, während im gelagerten Zitratplasma, das nicht mit EDTA versetzt wurde, die Thrombin- und thrombinartige Enzymaktivität weiter zunimmt und nach drei Monaten Lagerung bei -20° C das 5-10fache des Ausgangswertes beträgt.
Frisches Plasma, mit EDTA versetzt, bis eine Messketten-Spannung von 15 mV erreicht ist, weist bei dreimonatiger Lagerung bei -20° C nicht mehr als 1000 µE Thrombin pro mL auf. Die Bestimmung der Thrombinaktivität erfolgt mit chromogenem Substrat S-2238, wobei in diesem Fall sowohl Thrombin als auch thrombinartige Enzymaktivitäten gemessen und in Einheiten Thrombin zum Ausdruck gebracht werden.

### B. Proteasearmes, gelagertes Kryopräzipitat:

Aus dem mit EDTA versetzten Plasma, das tiefgefroren gelagert ist, kann beim Auftauen bei einer Temperatur, die 5° C nicht überschreitet, ein dabei entstander Niederschlag, das Kryopräzipitat, durch Zentrifugation zwischen 10000 und 20000 U/min gewonnen werden. Das Sediment wird durch Zusatz einer 0,1%igen Zitratlösung zu einem leicht flüssigen Brei aufgerührt, wobei die Temperatur zwischen 2° C und 4° C betragen soll, und kann in dieser Form zur weiteren Herstellung von Fibrinogen, Faktor VIII, Faktor V und Faktor XIII verwendet werden. Falls eine Lagerung erwünscht ist, wird zu dieser breiigen Suspension unter Rühren solange eine 1%ige EDTA-Lösung zugesetzt, bis eine daraus entnommene Probe, mit destilliertem Wasser um das 10fache verdünnt, der Kalziumionenaktivität einer 10µM Kalziumchloridlösung entspricht. Die so erhaltene EDTA-hältige, breiige Suspension wird dann eingefroren und bei -20° C oder tieferen Temperaturen gelagert. Nach Wiederauftauen der Kryopräzipitatsuspension innerhalb von drei Monaten beträgt die mit chromogenem Substrat S-2238 gemessene Thrombinaktivität in einer Lösung, die nicht mehr als 3 mg Fibrinogen pro mL enthält, 1000 µE Thrombin pro mL.

### C. Proteasearmer Kryopräzipitatüberstand:

Der nach dem Abzentrifugieren des Kryopräzipitates erhaltene Überstand kann als solcher sofort weiterverarbeitet werden. Bei vorgesehener Lagerung in tiefgefrorenem Zustand werden vorhandene Proteaseaktivitäten durch Zusatz von 1 E Heparin pro mL Kryopräzipitatüberstand minimiert. Für eine beabsichtigte Lagerzeit von sechs Monaten in tiefgefrorenem Zustand darf der mit Heparin versetzte Kryopräzipitatüberstand pro mL nicht mehr als 1 ng Faktor Xla, 100 E Kallikrein, 1 mE Faktor Xa, 10 mE Thrombin und 100 mE Plasmin enthalten.

### 2. Herstellung von thrombinfreiem Fibrinogen:

Etwa 650 g Kryopräzipitatpaste, die aus 100 L menschlichem Blutplasma gewonnen wurden, werden in 10 L einer 0,1%igen Natriumzitratlösung bei einer Temperatur von 2° bis 4° C unter Einhaltung eines pH-Wertes von zwischen 7,0 und 7,5 unter Rühren während einer Stunde gelöst. Die Lösung des Kryopräzipitats erfolgt nicht vollständig. Nach Entnahme einer Probe wird das Gesamtgewicht der Lösung bestimmt und die gesamte Lösung eingefroren und bei -20° C gelagert.

In der entnommenen Probe wird der Gehalt an Fibrinogen und Fibrinopeptid A bestimmt. Ebenso wird die Thrombinaktivität mit Hilfe von chromogenem Substrat S-2238 bei 4° C und bei 37° C gemessen. Der Fibrinogengehalt wird nephelometrisch und der Fibrinopeptid-A-Gehalt mit Hilfe einer Elisa-Methode quantitativ bestimmt.

Nach dem Auftauen der Hauptmenge wird, falls die Thrombinbestimmung bei 37° C eine Thrombinaktivität von mehr als 10 µE Thrombin/mL Fibrinogen ergeben hat, eine berechnete Menge r-Hirudin (Lepirudin) zugesetzt, die sich aus der in einem Vorversuch gemessenen Menge r-Hirudin ergibt, die erforderlich ist, um die Thrombinaktivität auf oder unter 10 µE Thrombin/mg Fibrinogen zu bringen. Die aufgetaute Kryopräzipitatlösung wird bei einer Temperatur, die 5° C nicht übersteigt, mit 150 g Glyzin pro kg Kryopräzipitatlösung versetzt und 30 Minuten gerührt. Das dabei ausgefällte Fibrinogen wird nach 12- bis 24-stündiger Lagerung zwischen 2° C und 4° C durch Zentrifugation in einer Sharples-Zentrifuge abgetrennt. Die Durchflusszeit wird so eingestellt, dass ein klarer, präzipitatfreier Überstand erhalten wird. Der Überstand kann zur weiteren Verarbeitung eingefroren und bei -20° C gelagert werden. In einer Probe des Überstandes werden noch vorhandenes Fibrinogen, Fibrinopeptid A, Thrombinaktivität und Hemmung der Thrombinaktivität bestimmt.

Das durch die Glyzinfällung und Zentrifugation gewonnene Sediment wird in einem 0,1%igem Zitratpuffer von pH 7,0 bis 7,5 gelöst, der Fibrinogengehalt nephelometrisch bestimmt und durch Zusatz von weiterem Zitratpuffer auf eine gewünschte Fibrinogenkonzentration von zwischen 1 % und 2% eingestellt. Die Lösung in Zitratpuffer wird bei einer Temperatur von 2° C bis 4° C vorgenommen, ebenso die nachfolgende Zentrifugation des gelösten Glyzinniederschlags bei 10000 bis 15000 Umdrehungen pro Minute und einer Zentrifugationsdauer von mindestens 10 Minuten. Das durch die Zentrifugation erhaltene Sediment wird verworfen und der Überstand mit 1/10 der ursprünglich zugesetzten Menge r-Hirudin versetzt und durch Zusatz von festem Glyzin auf eine Glyzinkonzentration von 15% eingestellt. Der pH-Wert der Suspension soll 6,5 nicht unterschreiten und nicht über 7,5 liegen. Erforderliche Korrekturen des pH-Werts werden mit einer 1%igen Zitronensäurelösung oder einer 0,1%igen Natronlauge durchgeführt. Die Fällung wird nach 12- bis 24-stündigem Stehen bei 2° C bis 4° C zentrifugiert und das erhaltene Fibrinogensediment in 0,1%iger Zitratlösung bei einem pH-Wert von 7,0 bis 7,5 gelöst. Falls die Lösung mehr als 1µE Thrombin pro mg Fibrinogen enthält, wird weiteres r-Hirudin zugesetzt, um die Thrombinaktivität unter 1µE Thrombin pro mg Fibrinogen zu bringen. Die Umfällung des Fibrinogens mit Glyzin und der allenfalls erforderliche Zusatz von r-Hirudin kann auch mehrfach wiederholt werden, um eine genügende Reinigung des Fibrinogens zu erzielen.

Ein genügend gereinigtes Fibrinogen liegt dann vor, wenn mindestens 95% des vorhandenen Proteins durch Thrombinzusatz gerinnen, der Fibrinogenpeak in der Gelfiltration mit Superose 6HR 10/30, Amersham, mindestens 94% beträgt und bei Auftragen von 10µg Fibrinogen in der SDS-Gelelektrophorese ein bandenreines Produkt vorliegt. Weiters darf bei der Verwendung eines reduzierenden Puffers kein Fibrinogenband auftreten, und es müssen drei Bande der entsprechenden Fibrinogenketten auferscheinen.

Die so erhaltene Fibrinogenlösung kann von überschüssigem Fällungsmittel durch Diafiltration gegen 0,1%iges Zitrat befreit werden, ebenso von Trinitrobutylphosphat und Tween-80 nach erfolgter Virusinaktivierung. Da die Diafiltration sowie die Virusinaktivierung mit Solvent/Detergent und eine allenfalls nachfolgende Nanofiltration bei Raumtemperatur durchgeführt werden, ist es allenfalls erforderlich, noch weitere Mengen r-Hirudin zuzusetzen, um die Thrombinaktivität während dieser Herstellungsstufen nicht über 1 µE Thrombin pro mg Fibrinogen ansteigen zu lassen.

Die virusinaktivierte und virusabgereicherte Fibrinogenlösung kann bis zur weiteren Verarbeitung tiefgefroren oder gefriergetrocknet gelagert werden. Nach Auftauen bzw. Rekonstitution des gefriergetrockneten Fibrinogens in einem geeigneten Lösungsmittel kann die Formulierung, Sterilfiltration, Portionierung und Verfüllung in Endverbrauchereinheiten vorgenommen werden. Das so hergestellte Fertigarzneimittel kann sowohl in flüssigem oder gefrorenem als auch gefriergetrockentem Zustand gelagert werden.

Die Integrität des so gewonnenen und zu einem fertigen Arzneimittel verarbeiteten Fibrinogens wird durch die während der gesamten Herstellung und Lagerung gebildete Menge Fibrinopeptid A bestimmt. Die Gesamtmenge des Fibrinopeptids A, bezogen auf die Gesamtausgangsmenge Fibrinogen, darf nicht mehr als 10%, und soll vorzugsweise 5%, betragen.

### 3. Herstellung von fibrinogenhältigen Lösungen, die bei Temperaturen zwischen 0° C und 5° C zu keiner Gelbildung führen:

Fibrinogen, nach konventionellen Methoden hergestellt, enthält zwischen 5% und 15% Fibrinmonomerkomplexe. Die genaue Bestimmung der Fibrinmonomerkomplexe erfolgt mit Hilfe der Gelfiltration und erfindungsgemäß durch einen Zusatz von 5% Harnstoff zu der zu untersuchenden fibrinogenhältigen Lösung. Durch den chaotropen Effekt des Harnstoffes wird eine gute Trennung von Fibrinmonomerkomplexen und Fibrin verursacht.

Eine Fibrinogenlösung, die zwischen 1 % und 10% Fibrinogen enthält, wird mit verschiedenen Mengen festen Arginins versetzt, sodass Fibrinogenlösungen entstehen, die 0,5%, 1%, 2%, 4% und 8% Arginin enthalten. Als Kontrolle wird eine Fibrinogenlösung ohne Zusatz von Arginin mitgeführt. Die Argininlösungen und die Kontrolle werden mindestens 15 Minuten bei Raumtemperatur gehalten und dann auf eine Temperatur zwischen 0° C und 5° C gebracht. Nach bis zu 24-stündiger Inkubation wird die Menge Arginin bestimmt, die zugesetzt werden muss, um eine Gelbildung zu verhindern.

Diese Vorgangsweise wird sowohl zur Herstellung einer Wirkstoffpräparation, die Fibrinogen als therapeutisches Protein enthält, als auch bei der Formulierung fibrinogenhältiger Arzneimittel verwendet, insbesondere wenn sie flüssig gelagert werden. Die ermittelte Menge Arginin kann aber auch vor dem Tiefrieren fibrinogenhältiger Arzneimittel zugesetzt werden, um nach Auftauen bzw. Rekonstitution mit Lösungsmitteln bei Lagerung im Kühlschrank eine Gelbildung zu verhindern.

### 4. Gewinnung fibrinolysefreier Thrombinlösungen:

Thrombin, nach einer beliebigen Methode durch Aktivierung von Prothrombin hergestellt, wird mehrfach (mindestens fünfmal) an CM-Sepharose absorbiert und mit einem Kochsalzgradienten, der mit einer 10 mM Phosphatlösung, pH 7,0, gepuffert ist, zwischen 120 und 180 mM Kochsalzlösung eluiert. Vor der Elution wird die thrombinhältige CM-Sepharose mit der 10fachen Menge ihres Volumens fünfmal gewaschen, wobei eine Waschlösung mit einem 10 mM Natriumphosphatgehalt und einem 50 mM Kochsalzgehalt bei pH 7,5 verwendet und der Waschvorgang bei zwischen 2° C bis 4° C durchgeführt wird.

Das mit zwischen 120 und 180 M Natriumchlorid eluierte Thrombin wird auf seinen Gehalt an Plasmin und mit t-PA aktiverbaren Plasminogen untersucht. Die Untersuchung erfolgt im Fibrinolysetest. Es werden 100 µL des Eluats, das auf etwa 100 E Thrombin pro mL eingestellt wurde, mit von 50 µL einer 0,025 molaren Kalziumchloridlösung und 100 µL einer 1%igen plasminogenfreien Fibrinogenlösung versetzt. Dieser Ansatz wird in einer Küvette zur Gerinnung gebracht und eine auftretende Lyse mit einem Elisa-Reader Sunrise TECAN über 18 Stunden beobachtet. Ein gleichzeitiger Ansatz, der auch noch 10 mE t-PA enthält, wird ebenfalls auf Lysezeit untersucht. Die zu untersuchende Thrombinlösung darf in beiden Ansätzen nach 18 Stunden keine Lyse verursachen.

Wird eine Lyse beobachtet, muss die beschriebene Reinigung von Thrombin an CM-Sepharose solange durchgeführt werden, bis in dem Fibrinolysetest keine Lyse des Ansatzes bis zu 18 Stunden beobachtet werden kann.

### 5. Herstellung stabiler, unschädlicher, funktionell und strukturell intakter Irtimunglobulin-G-Präparationen:

Cohn-Fraktion II, nach Entfernung von Alkohol durch Gefriertrocknung oder Diafiltration, wird durch Auflösen in destilliertem Wasser auf einen Gehalt von 1- 2% Protein gebracht. Diese Lösung wird mit ≤ 50 g Harnstoff pro L versetzt, mit 0,1%iger Natronlauge auf pH 8 gestellt, und soviel Natriumsulphat zugesetzt, bis eine gesättigte Natriumsulphatlösung entsteht. Die präzipitathältige Natriumsulphatlösung wird von einem allfälligen, ungelösten Überschuss von Natriumsulphat abdekantiert und das ausgefällte Immunglobulin durch Zentrifugation mit Hilfe einer Durchlaufzentrifuge gewonnen. Bei dem gesamten Vorgang soll der pH-Wert zwischen 7,5 und 8,0 liegen. Der durch Natriumsulphat-Sättigung erhaltene Immunglobulin-Niederschlag wird nach dem Zentrifugieren in destilliertem Wasser gelöst, wobei jene Menge Wasser verwendet wird, die eine etwa 5%ige Immunglobulin-Lösung ergibt. Diese Immunglobulinlösung wird auf ihren Gehalt an Plasmin und tPA oder Urokinase aktivierbarem Plasminogen untersucht. Zu diesem Zweck werden 500 µL dieser Lösung mit 10 E tPA oder Urokinase in einem Volumen von 50 µL versetzt und 50 µL Stimulator, der 1 % Fibrinopeptide enthält, zugesetzt. Nach Zugabe von 100 µL einer 4 mM chromogenen Substratlösung S-2403 wird der Ansatz bei 37° C inkubiert und die Menge p-Nitroanilin photometrisch bestimmt. Mit Hilfe des erhaltenen ΔA wird auf einer Standardeichkurve der Gehalt an Plasmineinheiten bestimmt, der nicht größer sein darf als 0,1 E pro mL einer 5%igen Immunglobulinlösung. Diese so bestimmte Menge Plasmin setzt sich aus bereits vorhandenen wie auch jenen Mengen Plasmin zusammen, die durch Plasminogenaktivatoren gebildetet wurden.

Die diafiltrierte Immunglobulinlösung darf auch nicht mehr als 10 E Kallikrein und nicht mehr als 1 ng Gerinnungsfaktor Xla enthalten, bezogen auf eine 5%ige Immunglobulinlösung. Der Gehalt an Kallikrein wird mit dem chromogenen Substrat S-2302 und der Gehalt an Faktor Xla mit dem chromogenen Substrat S-2366 bestimmt.

Um prokoagulante Wirkungen der erhaltenen Immunglobulin-G-Präparation auszuschließen, wird festgestellt, ob es zu einer Verkürzung der Gerinnungszeit eines rekalzifizierten Zitratplasmas kommt. Zu diesem Zweck werden 100 µL Zitratplasma mit 100 µL der zu untersuchenden Immunglobulin-Präparation mit 5% Proteingehalt gut vermischt und 100 µL einer 25 mM Kalziumchloridlösung zugesetzt und die Gerinnungszeit bei 37° C gemessen. Als Kontrolle dient ein Ansatz von 100 µL Zitratplasma mit 100 µL Puffer, dem nach gutem Durchmischen 100 µL einer mM Kalziumchloridlösung zugesetzt und in gleicher Weise die Gerinnungszeit bestimmt wird. Die in Abhängigkeit von dem verwendeten Zitratplasma gemessenen Gerinnungszeiten sollen zwischen 300 und 500 Sekunden liegen. Die Verkürzung der Gerinnungszeit durch ein 5%iges Immunglobulin soll weniger als 10% betragen.

### 6. Herstellung von Faktor-VIIIa-freiem Faktor-VIII-Konzentrat:

Eine tiefgefrorene Probe des tiefgefroren gelagerten Überstands der 15%igen Glyzinfällung des gelösten Kryopräzipitats wird nach dem Auftauen mit Hilfe des Faktor VIII Chromogen Kits, DADE Behring, auf die vorhandene, gesamte Aktivität an Faktor VIII untersucht, die sich aus dem vorhandenen Faktor VIII und VIIIa zusammensetzt. Die Bestimmung wird mit und ohne Zugabe des im Kit vorhandenen Thrombins durchgeführt. Die Differenz, die sich aus beiden Bestimmungen ergibt, wird als die Menge Faktor VIII bewertet, die sich durch Thrombin in Faktor VIIIa überführen lässt.

In der Probe wird auch die Menge des vorhandenen Thrombininhibitors in arbiträren Inhibitoreinheiten bestimmt. Falls in der untersuchten Probe weniger als 10 arbiträre Inhibitoreinheiten pro E Faktor VIII vorhanden sind, wird der Inhibitorgehalt durch Zugabe von r-Hirudin entsprechend erhöht.

5 mL der Probe werden durch Ultrafiltration mit Hilfe eines 30 K- oder 50 K-Dalton-Filters und Waschen mit einem 25 mM Phosphatpuffer pH 7,3, der 0,1% Zitrat enthält, von Glyzin weitgehend befreit und das Retentat auf Faktor-VIII-Spaltprodukte untersucht. Durch elektrophoretische Auftrennung der Proteine in einem 5%igen Acrylamidgel und in einem 10%igen Trenngel werden die geblotteten Proteinbande mit Hilfe eines monoklonalen Antikörpers gegen die schwere Kette des Faktors VIII auf allenfalls vorhandene Spaltprodukte untersucht.

Die eingefrorene Hauptmenge des Glyzinüberstandes aus Beispiel 2 wird unter Rühren bei 2° C bis 4° C pro kg Überstand mit 350 g β-Alanin versetzt. Der Niederschlag, der die Hauptmenge des Faktors VIII enthält, wird nach 12 bis 24 Stunden Stehen bei 2° C bis 4° C durch Zentrifugation bei einer Umdrehungszahl von zwischen 10000 und 15000 Umdrehungen pro Minute gewonnen, wobei die Durchlaufgeschwindigkeit so gewählt wird, dass ein klarer Überstand resultiert. Das Faktor-VIII-hältige Sediment wird in einer Menge 0,1%igem Zitratpuffer von pH 7,5 gelöst, sodass pro mL ein Faktor-VIII-Gehalt von zwischen 50 und 500 E resultiert. Der gesamt Vorgang wird bei einer Temperatur von zwischen 2° C bis 4° C durchgeführt. Nach Messung der Thrombin-Inhibitormenge wird, falls erforderlich, durch Zugabe von r-Hirudin das Verhältnis von Faktor VIII zu Inhibitor auf 10 arbiträre Thrombininhibitoreinheiten pro 1 E Faktor VIII eingestellt. Die Lösung wird dann bei Raumtemperatur durch Diafiltration gegen eine 0,1%ige Zitratlösung mit einem pH-Wert von 7,0 bis 7,5 von Glyzin und β-Alanin weitgehend befreit, wobei sie vor der Diafiltration durch ein oder zwei Umfällungen mit β-Alanin in Bezug auf Faktor VIII weiter gereinigt werden kann. Weitere Reinigungsschritte können bei Zimmertemperatur durch Chromatographie mit Hilfe von schwachen Anionenaustauschern durchgeführt werden. Ebenso kann die Virusinaktivierung mit Solvent/Detergent und eine nachfolgende, mehrstufige Nanofiltration zwischen 25° C und 30° C erfolgen. Eine Erhöhung der Faktor-VIII-Konzentration wird, falls erwünscht, durch Diafiltration erreicht. Das erhaltene Faktor-VIII-Konzentrat kann tiefgefroren oder nach Gefriertrocknung zwischengelagert werden. Die aufgetaute, gefrorene Lösung, bzw. das rekonstituierte, gefriergetrocknete Pulver wird formuliert, sterilfiltriert, portioniert abgefüllt und in den abgefüllten Fläschchen einer weiteren Gefriertrocknung unterzogen. Mindestens 80% der vorhandenen Faktor-VIII-Aktivität müssen als intakter Faktor VIII vorliegen, bis zu 20% derAktivität können durch Faktor VIIIa bedingt sein. In einer Lösung, die 100 E Faktor VIII enthält, darf keine Thrombinaktivität vorhanden sein, wobei eine Bestimmungsmethode verwendet wird, bei der noch 30 µE Thrombin pro mL bestimmbar sind.

### 7. Prothrombinkomplex-Präparationen frei von aktivierten Gerinnungsfaktoren:

1 L Kryopräzipitatüberstand wird mit 16 g A-50, einem schwachen Anionenaustauscher, versetzt, 15 Minuten bei Raumtemperatur gerührt, und der lonenaustauscher, der zu 90% oder mehr die im Kryoüberstand enthaltenen Gerinnungsfaktoren II, VII, IX und X absorbiert hat, durch Zentrifugation getrennt. Der Überstand, der die Hauptmenge des im Plasma enthaltenen Albumins bzw. der darin enthaltenen Immunglobuline enthält, wird zur Gewinnung von Inhibitoren, Immunglobulinen und Albumin weiterfraktioniert.

Der abgetrennte A-50 Anionenaustauscher wird dreimal mit je 5 L 150 mM Natriumchloridlösung bei Temperaturen von zwischen 2° C bis 4° C extensiv gewaschen, und dann wird mit Hilfe einer 500 mM Kochsalzlösung der aus den Gerinnungsfaktoren II, VII und X bestehende Prothrombinkomplex und ebenso die Hauptmenge des im Kryoüberstand enthaltenen Tissue-Factor-Pathway-Inhibitors (TFPI) eluiert. Der Vorgang der Absorption an und der Elution des schwachen Anionenaustauschers A-50 wird sooft wiederholt, bis im Faktor Xa Generationstest kein Faktor VIII pro 10 E Prothrombin bestimmbar wird. Die Prothrombinbestimmung erfolgt mit Hilfe von Ecarin und Messung des gebildeten Meizothrombins und Thrombins mit chromogenem Substrat S-2238. Die Bestimmung von TFPI erfolgt durch Messung der Inhibition der Faktor-Xa-Aktivität. Pro 1 E Prothrombin müssen mindestens 0,2 E TFPI vorhanden sein.

### 8. Stabilisierte therapeutische Proteine in Arzneimitteln:

Vor der Formulierung von Wirkstoffzubereitungen, die therapeutische Proteine enthalten, wird durch Probeformulierung festgestellt, ob noch Aktivitäten von Proteasen feststellbar sind, die Funktionen oder Strukturen therapeutischer Proteine zerstören. Wenn bei dem Reinigungsprozess therapeutischer Proteine die Abreinigung unvollständig ist, müssen bei der Formulierung von Arzneimitteln atoxische, virussichere, hochmolekulare Proteaseinhibitoren zugesetzt werden, die Proteasen soweit inaktivieren, dass sie zu keinem Verlust an Wirksamkeit und Sicherheit des Arzneimittels führen. Umgekehrt dürfen die zugesetzten Inhibitorenmengen die Wirksamkeit eines therapeutischen Proteins nicht verringern. Der Zusatz nicht-toxischer, hochmolekularer Proteaseinhibitoren, wie Antithrombin III und C₁-Inhibitor, alleine oder gemeinsam, erfolgt vorteilhafterweise mit unfraktioniertem Heparin oder HMW Heparin. Die zuzusetzende Menge Inhibitoren wird durch die erforderliche Minimierung von Enzymaktivitäten festgelegt. Wirkstoffzubereitungen, die therapeutische Proteine enthalten, werden mit den chromogenen Substraten S-2238, S-2302, S-2366, S-2403 und S-2765 auf ihre Spaltung durch Proteasen untersucht. Im Falle zu hoher Proteaseaktivitäten werden entsprechende Inhibitoren zur erforderlichen Absenkung der Enzymaktivität zugesetzt. Wenn aus den Voruntersuchungen hervorgeht, dass Enzymmengen, die mit chromogenen Substraten nicht mehr eindeutig bestimmbar sind, bereits zur Verminderung der Aktivität, Sicherheit und Stabilität von therapeutische Proteine enthaltenden Arzneimitteln, insbesondere bei längerer Lagerung, führen, werden die Aktivitätsbestimmungen mit den entsprechenden fluorogenen Substraten, die bis zu 100fach empfindlicher als chromogene Substrate sind, durchgeführt.

Die erforderlichen Zusätze an Enzyminhibitoren werden in einer Konzentrationsreihe ermittelt. Proben des zu formulierenden Arzneimittels werden in einer geometrischen Konzentrationsreihe mit Enzyminhibitoren vermischt und, bezogen auf 1 mL des gebrauchsfertigen Arzneimittels, von jedem chromogenen Substrat 100 nm zugesetzt. Diese Ansätze können je nach Erfordernis gefriergetrocknet, tiefgefroren bzw. flüssig bei Kühlschranktemperatur gelagert werden. Nach dreimonatiger Lagerung bei vorbestimmten Temperaturen werden die tiefgefrorenen und gefriergetrockneten Ansätze aufgetaut, bzw. rekonstituiert und in allen drei Ansatzreihen die gebildeten Mengen p-Nitroanilin photometrisch bestimmt. In entsprechenden Kontrollansätzen, die nur in Puffer gelöste, chromogene Substrate in einer 100 µM Konzentration enthalten, und in gleicher Art wie die Proben hergestellt und gelagert wurden, wird ebenfalls die Menge des gebildeten p-Nitroanilins bestimmt, und diese Mengen von den ermittelten Werten der Probeansätze in Abzug gebracht. Die Ansätze mit genügendem Inhibitorzusatz wie auch jene mit darüberliegenden Inhibitorkonzentrationen und ihre Kontrollen werden noch drei Tage bei Kühlschranktemperatur und 20 Stunden bei 25° C gelagert. Es werden jene Mengen Inhibitor bzw. Inhibitoren ermittelt, die die weitere Bildung von p-Nitroanilin verhindern.

Die gebildete Menge p-Nitroanilin nach einer Inkubation bei 37° C über 24 Stunden darf bei Verwendung von chromogenem Substrat S-2238 pro mL des gebrauchsfertigen Arzneimittels nicht mehr als 100 nm p-Nitroanilin betragen, bei Verwendung von S-2302 in gleicher Konzentration nicht mehr als 200 nm, bei chromogenem Substrat S-2366 nicht mehr als 50 nm, bei chromogenem Substrat S-2403 nicht mehr als 100 nm und bei chromogenem Substrat S-2765 nicht mehr als 100 nm betragen. Die entsprechenden Ansätze enthalten die chromogenen Substrate in einer Menge von 200 nm pro mL. Die zu untersuchenden Ausgangs-, Zwischen- und Endprodukte werden in en Mengen zugesetzt, dass nach einer Inkubation von 24 Stunden bei 37° C und einer Schichtdicke von 10 mm kein größerer Extinktionswert als 1,000 im Photometer abgelesen wird.

### 9. Prozess-Kontrollen bei der Herstellung thereapeutischer Proteine und Qualitätskontrollen von Wirkstoffzubereitungen, die therapeutische Proteine enthalten:

Die Enzymaktivitäten, die bei der Herstellung therapeutischer Proteine auftreten, stammen sowohl vom Ausgangsmaterial, als auch von Proteasen, die während des Herstellungsvorganges aus Zymogenen gebildet werden. Der gesamte Herstellungsvorgang wird unter Berücksichtigung der Proteasemengen, die sich im Ausgangsmaterial befinden, auf Aktivierung von Zymogenen zu Proteasen, Abreinigung solcher Proteasen und in den therapeutischen Proteinen noch verbliebene Proteaseaktivitäten überwacht. Es werden Thrombin, Kallikrein, Faktor Xla, Plasmin und Faktor Xa sowie thrombinartige, kallikreinartige, Faktor-Xla-artige, plasminartige und Faktor-Xa-artige Proteaseaktivitäten mit den chromogenen Substraten S-2238, S-2302, S-2366, S-2403 und S-2765 gemessen. Sämtliche hochmolekulare Substanzen, die sich im Ausgangsmaterial befinden und im Laufe der Herstellung von den therapeutischen Proteinen angetrennt werden, werden gesammelt und sofort tiefgefroren. Am Ende des Herstellungsverfahrens werden die gesammelten, tiefgefrorenen Rückstände aufgetaut, gepoolt und mit Hilfe der fünf chromogenen Substrate auf Enzymaktivität untersucht. Im Zusammenhang mit dem Enzymgehalt des Ausgangsmaterials kann abgeschätzt werden, in welchem Ausmaß die Aktivierung von Proenzymen während des Herstellungsverfahrens erfolgte. Wenn bei der Herstellung von therapeutischen Proteinen der Verdacht besteht, dass es durch die Anwesenheit von den aktivierten Gerinnungsfaktoren VII und IX in direkter oder indirekter Weise zu proteolytischen Einwirkungen auf ein bestimmtes therapeutisches Protein kommt, werden zusätzliche Bestimmungen solcher Enzymaktivitäten mit Spectrozym VIIa und IXa (American Diagnostics) vorgenommen. Bei therapeutischen Proteinen, die äußerst empfindlich auf Proteaseeinwirkungen reagieren, wie z.B. Faktor VIII, können zur Steigerung der Empfindlichkeit anstelle von chromogenen Substraten fluorogene Substrate herangezogen werden.

Die Menge einer bestimmten, in einer Probe vorhandenen Protease wird mit Hilfe einer Eichkurve vorgenommen. Die Eichkurven wurden für Thrombin mit dem NIBSC Standard 89/580, für Kallikrein mit Coachrom Humankallikrein 0,01 E/mL, erstellt. Die Eichkurve für Faktor Xla wurde mit einem Faktor-Xla-Konzentrat von American Diagnostics erstellt, wobei 260 ng Faktor Xla die Gerinnungszeit eines Faktor-XI-Mangelplasma zu 50% normalisieren. Für Plasmin wurde der 3. Internationale Standard 1998 von NIBSC 97/536 verwendet, für Faktor Xa eine Referenzpräparation von NIBSC. Für die Standardisierung der Faktor-Xla-Präparation wurde außerdem festgehalten, welche Menge von Faktor Xla in ng ausgedrückt, einer Menge von Faktor XI entspricht, die ein Faktor-XI-Mangelplasma zu 50% in seiner Gerinnungszeit normalisiert.

Falls eine entsprechende, erforderliche Reduktion von Proteaseaktivitäten durch Antithrombin III, C₁-Inhibitor und Heparin oder deren Kombination nicht erzielbar ist, können folgende Inhibitoren, allein oder gemeinsam, mit Antithrombin III, C₁-Inhibitor und Heparin verwendet werden: Aprotinin, Inter-α-Trypsin-Inhibitor, Plasma-Anti-Plasmin, Plasma-Anti-Trypsin und Tissue-Faktor-Pathway-Inhibitor. Alle diese Inhibitoren müssen virussicher sein.

Wirkstoffzubereitungen, die aktive pharmazeutische Substanzen (ICH: drug substances) enthalten, sind so vorformuliert, dass Aktivität, Unschädlichkeit und Stabilität bei entsprechender Lagerung über Wochen bis Monate erhalten bleiben. Die erforderlichen Qualitätskontrollen beziehen sich im Wesentlichen auf die volle Erhaltung der pharmazeutischen Aktivität und auf fehlende Prokoagulationsaktivität. Um für eine Wirkstoffzubereitung die maximale Lagerzeit zu ermitteln, wird die Zeit bestimmt, in der noch keine Abnahme der pharmazeutischen Aktivität, keine messbare Zunahme der Proteaseaktivität und keine mehr als 10%igen Verkürzungen der Plasmaprothrombinzeit, der PlasmaaPTT und Plasmarekalzifizierungszeit auftreten. Wenn Wirkstoffzubereitungen tiefgefroren gelagert werden und in zu kurzer Zeit ihre Lagerfähigkeit erlischt, so kann durch Gefriertrocknung und nötigenfalls Lagerung des gefriergetrockneten Wirkstoffes bei -20° C die Lagerfähigkeit wesentlich erhöht werden.

### 10.Abtrennung von Enzymen und Viren sowie Inaktivierung von Viren und Inhibition von Enzymen in zusätzlichen Herstellungsstufen:

Bei verschiedenen Herstellungsprozessen von therapeutischen Proteinen kann es zur Aktivierung eines oder mehrerer Proenzyme kommen. Solche Enzyme können therapeutische Proteine nachhaltig verändern. Die Wirkung mancher Enzyme kann stark vom Redoxpotenzial abhängig sein. Um die fördernde Wirkung von Faktor XIIIa auf die Bildung von Fibrinmonomerkomplexe zu verhindern, kann durch Zusatz von Cystin die enzymatische Aktivität von Faktor Xllla weitgehend reduziert werden. Umgekehrt wird durch Cystein und andere Reduktionsmittel die Aktivität von Faktor XIII stark gesteigert. Da Faktor XIII schon in geringer Konzentration zur vermehrten Bildung von Fibrinmonomerkomplexen führen kann, ist eine entsprechend empfindliche Bestimmungsmethode erforderlich.

Das nach dem Beispiel 2 hergestellte, thrombinfreie Fibrinogen wird zur Entfernung von Faktor XIII und Faktor Xllla in einer etwa 1%igen Lösung mit Cystin versetzt, bis die Cystinkonzentration eine Molarität von 50 mM erreicht. Fibrinogen wird aus dieser Lösung durch Zugabe von Glyzin (12%) ausgefällt, der Fibrinogenniederschlag durch Zentrifugation gewonnen, und der Überstand verworfen. Dieser Vorgang wird 2- bis 4-mal wiederholt, indem der Niederschlag in einem 50 mM Cystinpuffer mit 0,1% Zitrat bei pH 7,0 gelöst und die Fällung mit Glyzin wiederholt wird. Die Fällung von Fibrinogen wird sooft wiederholt, bis der Faktor-XIII-Gehalt nicht mehr als 10 mE pro mL einer 1%igen Fibrinogenlösung beträgt.

Wie in Beispiel 2 gezeigt wurde, kann durch Verwendung von Aminosäuren in hoher Konzentration bei der Herstellung von Fibrinogen eine proteolytische Spaltung weitgehend verhindert werden. Wie ebenfalls in Beispiel 2 gezeigt wurde, gelingt es, schädliche Thrombinaktivitäten durch avide, hochmolekulare Inhibitoren weitgehend auszuschalten. Der Zusatz von anorganischen Salzen mit chaotropen Agenzien fördert einerseits die Aufspaltung von Enzymsubstratbindungen und inhibiert dadurch die enzymatische Aktivität und ermöglicht gleichzeitig eine bessere Abtrennung der Enzyme von ihren Substraten. Für manche enzymatische Einwirkung auf hochmolekulare Substrate ist deren dreidimensionale Konfiguration, die durch einen spezifischen Salzgehalt in einem bestimmten Konzentrationsbereich bestimmt wird, erforderlich. Durch Komplexbildner wie EDTA kann diese Substratkonfiguration reversibel verändert und dadurch die Einwirkung verschiedener Enzyme auf ihre Substrate stark vermindert werden.

Virusinaktivierungen, z.B. mit Detergenzien, müssen über längere Zeit im Stundenbereich durchgeführt werden, ebenso bei Raumtemperatur oder leicht darüber liegender Temperatur. Therapeutische Proteine, falls sie ungenügend von Proteasen oder von solchen Enzymsystemen, die Proteasen bilden können, befreit sind, müssen mit genügenden Mengen Proteaseinhibitoren versetzt werden, damit bei der Virusinaktivierung keine proteolytische Schädigung eines therapeutischen Proteins erfolgt. In Beispiel 2 wird dies durch Zusatz von r-Hirudin erreicht. Ebenso kann dies mit Hilfe von Antithrombin III und Heparin in einem Verhältnis von 1 E Antithrombin mit 6 E Heparin erfolgen.

Zur Abreicherung von Viren haben sich Nanofilter als besonders wirksam erwiesen. Die Nanofiltration, insbesondere bei Filtern mit sehr kleinen Porenweiten wie 20nm und 15 nm, geht bei Raumtemperatur langsam vor sich. Durch Temperaturerhöhungen bis auf 40° C kann die Geschwindigkeit der Filtration wesentlich gesteigert werden. Da das Temperaturoptimum der meisten Proteasen zwischen 35° C und 40° C liegt, wird in einer Probe der zu nanofiltrierenden Lösung eines therapeutischen Proteins bestimmt, welche Mengen von Proteaseinhibitoren zugesetzt werden müssen, damit das therapeutische Protein nicht enzymatisch geschädigt wird.

So werden Prothrombinkomplex-Konzentrate durch Verdünnung mit 0,1%igem Zitratpuffer bei pH 7,0 auf einen Proteingehalt von 5 mg pro mL eingestellt und durch Filter mit Porenweiten von 220 nm, 75 nm und 35 nm bei Raumtemperatur gefiltriert. Das Filtrat der 35 nm-Filtration wird sterilfiltriert und unter aseptischen Bedingungen bei 40° C± 2° C durch Filter mit Porenweiten von 20 nm und 15 nm (Ashai) unter aseptischen Bedingungen filtriert. Das 15 nm-Filtrat wird bei Kühlschranktemperatur ultrafiltriert. Das so erhaltene ProthrombinkomplexKonzentrat muss pyrogenfrei sein und pro 100 E Prothrombin mindestens 5 E TFPI enthalten. Die Enzymaktivität des Konzentrats darf pro 100 E, gemessen mit chromogenen Substraten, nicht mehr als 0,1 E Thrombin, 0,1 E Plasmin, 100 ng Faktor Xla, 0,2 E Faktor Xa und 0,05 E Kallikrein betragen.

### 11. Ultrasensitive Bestimmung von Thrombin:

Durch entsprechende Langzeitinkubation eines Enzyms mit einem geeigneten Substrat bei seinem Temperaturoptimum und optimalem pH-Wert kann eine wesentliche Steigerung der Empfindlichkeit der Bestimmung der Enzymaktivität erreicht werden. Bei einer Langzeitinkubation ist es erforderlich, sowohl das Enzym als auch das Substrat zu stabilisieren. Dies wird dadurch erreicht, dass die Bestimmung der Thrombinaktivität in einer Lösung durchgeführt wird, die 1 % Polyäthylenglykol mit einem Molekulargewicht von zwischen 6000 bis 8000 enthält. Die Inkubationszeit kann mit 15 Stunden festgelegt, und es können Proben aus einem Thrombinansatz nach 3 Minuten, 10 Minuten, 30 Minuten, 1 1/2 Stunden, 5 Stunden und 15 Stunden entnommen werden. Je nach verwendetem Substrat kann die Thrombinbestimmung noch im Bereich von Mikroeinheiten (µE) bis Nanoeinheiten (nE) vorgenommen werden.

Um die Thrombinaktivität während der Zwischenlagerung von Intermediärprodukten bzw. Fertigarzneimitteln, die thrombinempfindliche, therapeutische Proteine enthalten, zu messen, kann die Inkubationszeit auch wesentlich verlängert und die Temperatur entsprechend der erforderlichen Lagertemperatur verändert werden. Diese Methode ist auch geeignet, akzelerierte Stabilitätsuntersuchungen durchzuführen und die Eignung von bestimmten Thrombininhibitoren zu untersuchen. Ein entsprechender Versuchsansatz kann wie folgt durchgeführt werden:

Aus einer zu untersuchenden Probe wird eine geometrische Verdünnungsreihe mit einem 50 mM Trispuffer pH 8,3 hergestellt, der noch 1% PEG (Molekulargewicht 6000 bis 8000) und NaCl in einer 100 mM Konzentration enthält. Dieser Puffer wird auch zur Herstellung einer 400 µM Lösung von dem chromogenen Substrat S-2238 verwendet, und es werden 500 µL der S-2238-Lösung zu jeder der Probeverdünnungen zugesetzt. Als Kontrolle werden 500 µL der S-2238-Lösung, der 500 µL Trispuffer zugesetzt wurden, verwendet. Diese Ansätze können bei den gewünschten Temperaturen (0° C bis 45° C) bis zu mehreren Wochen inkubiert werden. Gespaltenes Substrat S-2238 wird photometrisch gemessen. Von den abgelesenen Extinktionswerten wird der Extinktionswert der Kontrolle abgezogen und der Differenzwert mit Hilfe einer Standardkurve zur Berechnung der Enzymaktivität verwendet.

### 12. Bestimmung der Integrität von Fibrinogen durch Quantitierung des Fibrinopeptids A:

Intaktes, reines Fibrinogen enthält weniger als 1 o/oo freies Fibrinopeptid A. Fibrinogen wird durch die Protease Thrombin proteolytisch in Fibrinopeptid A und Fibrinopeptid B, die je ein Molekulargewicht von etwa 1500 besitzen, und das Fibrinmonomer mit einem Molekulargewicht von etwa 330000 gespalten. Fibrinmonomere werden von Fibrinopeptiden mit 75%igem Äthanol bei Raumtemperatur abgetrennt. Das dabei ausgefällte Fibrinogen und Fibrinmonomer wird durch Zentrifugation von den sich im Überstand befindlichen Fibrinopeptiden abgetrennt und der Überstand zur vollkommenen Entfernung von Fibrinogen und Fibrinmonomer mit einer Bentonitsuspension in 75%igem Äthanol versetzt. Bentonit wird durch Zentrifugation abgetrennt, der Überstand quantitativ in einen Verdampfungskolben übergeführt, das Bentonitsediment mit 75%igem Äthanol aufgeschwemmt, zentrifugiert und der Überstand ebenfalls in den Verdampfungskolben gebracht. Die Lösung wird im Verdampfungskolben im Vakuum zur Trockene verdampft, der Rückstand in einer gemessenen Menge Puffer aufgenommen und der Gehalt an Fibrinopeptid A mit Hilfe eines Elisa-Testkits bestimmt. Die in diesem Testkit beschriebene Abtrennung von Fibrin und Fibrinmonomeren durch Bentonit hat sich als nicht ausreichend erwiesen (Novitec®, HiSS Diagnostics GmbH, Freiburg).

Bei einer kompletten Spaltung von Fibrinogen durch Thrombin bilden sich aus 1000000 ng Fibrinogen etwa 5000 ng Fibrinopeptid A. Wenn nur 1 % des vorliegenden Fibrinogens gespalten wird, werden dementsprechend nur 50 ng Fibrinopeptid A gebildet. Mit Hilfe eines entsprechend empfindlichen Elisa-Tests ist es möglich, noch 1 ng Fibrinopeptid A pro mL zu bestimmen, sodass dadurch eine Möglichkeit besteht, die Fibrinogenspaltung durch Thrombin über die gesamte Herstellungs- und Lagerperiode des therapeutischen Proteins Fibrinogen hinreichend genau zu bestimmen.

Zur Durchführung der Bestimmung werden 500 µL der zu untersuchenden fibrinogenhältigen Lösung, die einen genau bestimmten Fibrinogengehalt, der über 1 mg Fibrinogen pro mL liegen muss, mit 1500 µL absolutem Alkohol vermischt, 10 Minuten bei Raumtemperatur gehalten, und 3 Minuten zwischen 3000 und 5000 Umdrehungen pro Minute zentrifugiert. 1 mL des Überstandes wird mit 2 mL einer alkoholischen Bentonitsuspension versetzt, die aus 1 mL der im Kit vorhandenen Bentonitsuspension mit 3 mL absolutem Alkohol erhalten wurde. Dieses Gemisch wird nach wiederholtem, starkem Schütteln nach 10 Minuten zentrifugiert. 1 mL des Überstandes wird in einem Vakuumverdampfer getrocknet und der Trockenrückstand in 250 µL Trispuffer pH 7,0 gelöst. 100 µL dieser Lösung werden für den Elisa-Test eingesetzt und der Gehalt an Fibrinopeptid A in ng an einer vorher erstellten Standardkurve ermittelt. Die Standardkurve wird wie folgt erhalten:
Fibrinopeptid A (Sigma) wird mit Trispuffer gelöst und durch entsprechende Verdünnungen Fibrinopeptid-A-Lösungen von 1, 2, 5, 10 und 20 ng Fibrinopeptid A pro mL erhalten. Je 100 µL dieser Verdünnungen werden in dem Elisa-Test eingesetzt.
Dieser Test wird als kompetitives Enzyme-Linked Immunosorbent Assay (Elisa) durchgeführt. 100 µL der von Proteinen befreiten, Fibrinopeptid-A-hältigen Proben werden eine Stunde mit 100 µL eines anti-Fibrinopeptid-A-Antikörpers bei Raumtemperatur in Mikrotiterplatten gehalten, deren Wells mit einem anti-IGg-Antikörper vorbehandelt sind. Zu jedem der Wells werden 100 µL Fibrinopeptid-A-Biotin-Konjugat zugesetzt und eine weitere Stunde bei Raumtemperatur inkubiert. Nach Waschen der Wells der Mikrotiterplatten werden zu jedem der Wells 100 µL Streptavidin-Peroxydase-Konjugat zugesetzt, und nach 30-minütigem Stehen bei Raumtemperatur werden die Wells der Mikrotiterplatten einer weiteren Waschung unterzogen. Zu jedem der Wells wird Enzymsubstrat TMB zugesetzt, und es wird 30 Minuten bei Zimmertemperatur inkubiert. Darnach wird die Farbentwicklung mit 100 µL 1 M Salzsäure gestoppt und die Extinktion bei 450 nm gemessen. Die abgelesenen Extinktionen sind umgekehrt proportional zur Fibrinopeptid-A-Konzentration der Proben, deren Gehalt an Fibrinopeptid A mit Hilfe einer Referenzkurve bestimmt wird.

### 13. Bestimmung der Enzym-Inhibitorkonzentration.

Antithrombinbestimmung:
Das Prinzip der Bestimmung ist die Messung einer Inhibitormenge, die 1000µE Thrombin in 1 mL Puffervolumen in Anwesenheit einer 200 µM Konzentration von chromogenem Substrat S-2238 bei einer Inkubationsdauer von 24 Stunden und 37° C um 50% reduziert.

Zur Bestimmung der Antithrombinmenge werden Verdünnungen des Inhibitors in 1%igem PEG zu einer entsprechenden Thrombin-Substratmischung zugefügt und mit Trispuffer auf 1 mL aufgefüllt. Die Kinetik der noch vorhandenen Thrombinaktivität wird mindestens zu vier Zeitpunkten innerhalb des 24-Stundenintervalls bei einer Temperatur von 37° C gemessen. Aus einer vorher erstellten Eichkurve wird jene Menge Inhibitor bestimmt, die 50% der Aktivität von 1000 µE Thrombin hemmt. Diese Inhibitormenge wird arbiträr als 1 Antithrombineinheit festgelegt.

Das verwendete r-Hirudin von Hoechst Marion Roussel wurde als Fertigarzneimittel unter dem Handelsnamen Refludan verwendet und auf seinen Gehalt an arbiträren Inhibitoreinheiten untersucht.

Unter Verwendung eines Trispuffers von pH 8,3 und den Zusätzen wie in Beispiel 1 angegeben, wird eine geometrische Verdünnungsreihe des r-Hirudins hergestellt, die r-Hirudin in Konzentrationen von 1 bis 16 ng pro mL enthält. 500 µL jeder dieser Hirudinlösungen wird mit 250 µL einer 800 µM Lösung von chromogenem Substrat S-2238 und 250 µL einer Thrombinlösung mit 4 mE Thrombin versetzt und 24 Stunden bei 37° C inkubiert. Eine Kontrolle, die nur Thrombin in einer Konzentration von 1000 µE in einer 200 µM chromogenen Substratlösung S-2238 enthält, wird ebenfalls 24 Stunden lang bei 37° C inkubiert. Als weitere Kontrolle dient ein 24-stündiger Inkubationsansatz von 200 µM S-2238.

Nach 24-stündiger Inkubation wird die Extinktion der einzelnen Ansätze bei 405 nm gemessen und von den gemessenen Extinktionen der Extinktionswert der Kontrolle, die nur chromogenes Substrat enthält, abgezogen. Der Extinktionswert der Thrombinlösung, die nur 1000 µE Thrombin pro mL enthält, von der der Leerwert abgezogen wurde, wird als 100% der vorhandenen Thrombinaktivität angesetzt und jene Menge Hirudin ermittelt, die 50% dieser Thrombinaktivität inhibiert.

### Literatur

Andersson LO, Forsman N, Huang K, Larsen K, Lundin A, Pavlu B, Sandberg H, Sewerin K, Smart J. Isolation and characterization of human factor VIII: Molecular forms in commercial factor VIII concentrate, cryoprecipitate, and plasma. Proc Natl Acad Sci 1986; 83:2979-2983.
Brummel KE, Butenas S, Mann KG. An Integrated Study of Fibrinogen during Blood Coagulation. J Biol Chem 1999; 274:22862-22870.
Eaton D, Rodriguez H, Vehar GA. Proteolytic Processing of Human Factor VIII. Correlation of Specific Cleavages by Thrombin, Factor Xa, and Activated Protein C with Activation and Inactivation of Factor VIII Coagulant Activity. Biochemistry 1986; 25:505-512.
Hemker HC. Thrombin Generation in a Reconstituted System: A Comment. Thromb Haemost 2002; 87:551-552.
Johnson AJ, Fulton AJ. Antihemophilic Factor Stabilization. United States Patents 1994 and 1996; Patent Numbers 5,278,289 and 5,484,890.
Kunicki TJ, Montgomery RR. Method for Maintaining Intact, Non-Degraded Factor VIII/Von-Willebrand Factor During Blood Processing. United States Patents 1987 and 1992; Patent Numbers 4,710,381 and 5,149,787.
Lawson JH, Kalafatis M, Stram S, Mann KG. A Model for the Tissue Factor Pathway to Thrombin. J Biol Chem 1994; 269:23357-23366.
Mann KG, Butenas S. Thrombin Generation in a Reconstituted System: A Reply. Thromb Haemost 2002; 87:552-554.
Mutch NJ, Robbie LA, Booth NA. Human Thrombi Contain an Abundance of Active Thrombin. Thromb Haemost 2001; 86:1028-1034.
Rotblat F, O'Brien DP, O'Brien FJ, Goodall AH, Tuddenham EGD. Purification of Human Factor VIII:C and Its Characterization by Western Blotting Using Monoclonal Antibodies. Biochemistry 1985; 24:4294-4300.
Siebenlist KR, Meh DA, Mosesson MW. Protransglutaminase (Factor XIII) Mediated Crosslinking of Fibrinogen and Fibrin. Thromb Haemost 2001; 86:1221-1228.

## Patentansprüche

1. Aus virussicheren, pharmazeutischen Wirkstoffzubereitungen hergestellte, lagerfähige Arzneimittel, die ein oder mehrere aus Plasma oder gentechnologisch gewonnene, intakte, therapeutische Proteine als aktive pharmazeutische Substanz bzw. Substanzen enthalten, wobei in den Wirkstoffzubereitungen weder freie, noch an ihre Substrate gebundene, aktive Enzyme, insbesondere Proteasen, vorhanden sind, die gegen das bzw. die vorhandenen therapeutischen Proteine wirken.

2. Wie Anspruch 1, indem die Wirkstoffzubereitungen auch keine aktiven Proteasen oder Proteasekaskaden enthalten, die die Zymogene der in Anspruch 1 bezeichneten Enzyme aktivieren.

3. Wie Ansprüche 1 und 2, indem auch keine hochmolekularen Kofaktoren oder Prokofaktoren in den Wirkstoffzubereitungen enthalten sind, die direkt oder indirekt die Einwirkung von Enzymen auf die darin enthaltenen therapeutischen Proteine fördern.

4. Wirkstoffzubereitungen wie in Ansprüchen 1 bis 3, die einen oder mehrere atoxische, virussichere, hochmolekulare, vorzugsweise allogene Proteaseinhibitoren enthalten und die die Aktivierung von Zymogenen durch Proteasen oder durch Proteasekaskaden und ebenso die proteolytische Wirkung der aus den Zymogenen gebildeten, gegen das, bzw. die in den Wirkstoffzubereitungen vorhandene(n), therapeutische(n) Protein(e) wirkenden Proteasen hemmen.

5. Therapeutische Proteine zur Herstellung von Wirkstoffzubereitungen nach Ansprüchen 1 bis 4, die ein oder mehrere atoxische, virussichere, hochmolekulare Enzyminhibitoren in einer Endkonzentration enthalten, die die Wirkung der therapeutischen Proteine nicht beeinflusst.

6. Reinigungsverfahren zur Gewinnung therapeutischer Proteine aus Blut oder Biomassen, um auf diese Proteine einwirkende Enzyme, insbesondere Proteasen, zu entfernen und deren enzymatische Wirkung während aller Stufen im Reinigungsprozess sowie bei der Lagerung von Ausgangsmaterialien, Zwischen- und Endprodukten dadurch so nieder als möglich zu halten, dass eine Temperatur von 10° C nicht überschritten wird, enzymaktivierende Ionen durch Komplexbildung unwirksam gemacht und/oder enzymhemmende, anorganische und organische Ionen und Zwitterionen zugesetzt und/oder Redoxpotenziale mit atoxischen Reduktions- oder Oxydationsmitteln so eingestellt werden, dass am Ende des Herstellungsprozesses mindestens 60%, vorzugsweise 95%, der therapeutischen Proteine funktionell voll erhalten sind, wobei die zugesetzten Hilfsmaterialien wieder entfernt werden.

7. Wie Anspruch 5, indem zusätzlich atoxische, chaotrope Substanzen eingesetzt werden, die eine Enzym-Substratbindung dissoziieren bzw. eine solche Bindung nicht zustande kommen lassen und dadurch die Trennung von an therapeutische Proteine gebundenen Enzymen, insbesondere Proteasen, verbessern, und die chaotropen Substanzen nach dem Trennverfahren wieder entfernt werden.

8. Verfahren zur Virusinaktivierung von therapeutischen Proteinen nach Ansprüchen 5 bis 7, die frei von aktiven, gegen therapeutische Proteine wirkenden Enzymen sind, wobei zur Virusinaktivierung viruzide Chemikalien bei Zimmertemperatur oder erhöhten Temperaturen zugesetzt und diese nach Beendigung des Virusinaktivierungsverfahrens wieder von den therapeutischen Proteinen abgetrennt werden.

9. Ein Verfahren nach Ansprüchen 5 bis 8, wodurch enzymfreie Lösungen von therapeutischen Proteinen durch Nanofiltration bei Temperaturen zwischen 20° und 40° C unter Verwendung von Nanofiltern mit einer Porenweite von 75 nm und 35 nm sowie vorzugsweise einer zusätzlichen Nanofiltration durch Filter mit Porenweiten von 20 nm und/oder 15 nm virusabgereichert werden.

10. Verfahren zur Reinigung und zur Entfernung von Viren nach Ansprüchen 8 und 9, indem durch Einhaltung einer Minimalkonzentration von erforderlichenfalls virussicheren, hochmolekularen, vorzugsweise allogenen Enzyminhibitoren auch noch Restaktivitäten von Enzymen in allen Zwischenprodukten bei ihrer Herstellung und Lagerung gehemmt werden, und die hochmolekularen Inhibitoren nachfolgend soweit entfernt werden, dass die Wirkung der therapeutischen Proteine bei ihrer Anwendung nicht beeinträchtigt wird.

11. Plasma zur Gewinnung von zur Herstellung therapeutischer Proteine als Wirksubstanzen in Arzneimitteln geeigneter Rohfraktionen, das sofort nach seiner Abtrennung von Blut mit atoxischen Komplexbildnern und/oder Natriumzitrat versetzt wird, bis die Kalziumionenaktivität unter die Ionenaktivität einer 10 µM Kalziumchloridlösung gesunken ist und dieses Plasma als solches weiter verarbeitet oder bei -20°C gelagert wird.

12. Kryopräzipitat, das nach Anspruch 11 gewonnen wird und zu einer viskosen Suspension mit einem Gehalt von etwa 3% Fibrinogen durch Zugabe von Wasser für Injektionszwecke verrührt und durch Zugabe von Komplexbildnern und/oder Natriumzitrat auf eine Kalziumionenaktivität eingestellt wird, die einer 10 µM Kalziumchloridlösung entspricht.

13. Kryopräzipitations-Überstand aus Plasma, das nach Anspruch 11 gewonnen und von Kryopräzipitat durch Zentrifugation abgetrennt wird und nach Zugabe von atoxischen Proteaseinhibitoren weniger als je 0,01 E Faktor IIa, VIIa, Xa, Xla, Kallikrein, Plasminogenaktivator und Plasmin enthält.

14. Virussicheres Fibrinogen als aktive pharmazeutische Substanz in Arzneimitteln, **dadurch gekennzeichnet, dass** es weniger als 4% Fibrinmonomerkomplexe bezogen auf gerinnbares Protein und weniger als 1 µE Thrombin pro mg Fibrinogen enthält.

15. In flüssigem Zustand lagerfähiges, fibrinogenhältiges Arzneimittel, **dadurch gekennzeichnet, dass** es eine Thrombinaktivität von weniger als 1 µE Thrombin pro mg Fibrinogen enthält und durch Zusatz von atoxischen, chaotropen Agenzien bei Kühlschranktemperatur zwischen 2° C und 8° C ohne Gelbildung gelagert werden kann.

16. Gereinigtes Thrombin, frei von Plasmin und anderen fibrinolytischen Proteasen, das bei einem Mischungsverhältnis von 500 E Thrombin mit 3 mg Fibrinogen Fibrin bildet, das mindestens 10 Stunden bei Raumtemperatur keine Lyse aufweist.

17. Lösungen von Immunglobulin G, die weniger als 100 µE Plasmin pro mg Immunglobulin und keine in einer 10%igen Immunglobulin-Lösung nachweisbaren Aktivitäten von Kallikrein, Faktor Xla und Faktor XIIa enthalten.

18. Virussischere Faktor-VIII-Zubereitung, **dadurch gekennzeichnet, dass** sie keine nachweisbare Thrombinaktivität und einen Antithrombingehalt von weniger als 10 arbiträren Antithrombineinheiten pro Einheit Faktor VIII aufweist und dass Faktor VIII zu maximal 20% in aktivierter Form (FVIIIa) vorliegt.

19. Gerinnungsfaktoren II-, VII-, IX- und X-hältige Arzneimittel, die pro 100 E Gerinnungsfaktor II weniger als 100 µE Thrombin, pro 100 E Faktor VII weniger als 1 mE Faktor Vlla, pro 100 E Faktor IX weniger als 1 mE Faktor IXa und pro 100 E Faktor X weniger als 100 µE Faktor Xa aufweisen.

20. Methoden, Reagenzien oder zusammengesetzte Reagenzien, mit deren Hilfe bei einer Langzeitinkubation zwischen 10 und 1000 Stunden, vorzugsweise 30 bis 100 Stunden, Enzyme auch in Anwesenheit ihrer natürlichen Substrate in piko-und femtomolaren Konzentrationen bei Temperaturen zwischen 0° C und 45° C nachgewiesen und bestimmt werden können.

21. Verfahren zum Nachweis und zur Bestimmung von durch Enzyme in mikro- bis femtomolaren Konzentrationen verursachten enzymatischen Umsatzprodukten in nach den Methoden in Ansprüchen 1 bis 10 hergestellten therapeutischen Proteinen durch Massenspektrometrie, HPLC, Gelfiltration und chromatographische, elektrophoretische oder immunologische Methoden.

22. Verfahren nach Anspruch 16, wobei der analytische Nachweis und die Bestimmung von enzymatischen Aktivitäten mit Hilfe von chromogenen oder fluorogenen Substraten durch Zusatz chaotroper Agenzien verbessert wird.

23. Bestimmung von Enzymaktivitäten nach den Ansprüchen 11, 13 und 15 in Proben zur Prozess-Kontrolle.

24. Bestimmung von Enzymaktivitäten nach den Ansprüchen 11, 13 und 15 zur Erreichung einer optimalen Stabilität von Endprodukten durch entsprechende Formulierung und Selektion optimaler Lagerbedingungen.
